(19) Europäisches Patentamt
European Patent Office
Office européen des brevets

(11) EP 0 842 135 B1

(12) # FASCICULE DE BREVET EUROPEEN

(45) Date de publication et mention de la délivrance du brevet:
**13.10.1999 Bulletin 1999/41**

(21) Numéro de dépôt: **96924919.2**

(22) Date de dépôt: **02.07.1996**

(51) Int Cl.[6]: **C07C 19/075**, C07C 17/16, C07C 17/093, C07C 29/64

(86) Numéro de dépôt international:
**PCT/FR96/01027**

(87) Numéro de publication internationale:
**WO 97/03036 (30.01.1997 Gazette 1997/06)**

(54) **PROCEDE DE PREPARATION D'ALPHA, OMEGA-BROMOCHLOROALCANES**

VERFAHREN ZUR HERSTELLUNG VON ALPHA, OMEGA-BROMOCHLOROALKANEN

METHOD FOR PREPARING ALPHA, OMEGA-BROMOCHLOROALKANES

(84) Etats contractants désignés:
**AT BE CH DE DK ES FR GB IE IT LI NL**

(30) Priorité: **11.07.1995 FR 9508360**

(43) Date de publication de la demande:
**20.05.1998 Bulletin 1998/21**

(73) Titulaire: **ELF ATOCHEM S.A.**
**92800 Puteaux, Hauts-de-Seine (FR)**

(72) Inventeurs:
- **DRIVON, Gilles**
  **F-69850 Saint-Martin-en-Haut (FR)**
- **RUPPIN, Christophe**
  **F-69310 Pierre-Bénite (FR)**

(56) Documents cités:
**FR-A- 1 093 998** **US-A- 2 868 850**

- **JOURNAL OF THE AMERICAN CHEMICAL SOCIETY, vol. 71, DC US, pages 1292-1297, XP002015946 M.S. NEWMAN ET AL.: "The Preparation of the Six n-Octynoic Acids"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 842 135 B1

## Description

La présente invention concerne un procédé de préparation directe d'$\alpha$,$\omega$-bromochloroalcanes à partir d'ethers-oxydes (éthers) cycliques.

Les halogénoalcanes et, plus particulièrement les $\alpha$,$\omega$-bromochloroalcanes sont largement utilisés comme matières premières pour la préparation de produits pharmaceutiques, de pesticides et de détergents.

De nombreuses méthodes ont été décrites pour obtenir ces $\alpha$,$\omega$-bromochloroalcanes.

Le plus souvent, elles font appel à la réaction des halogènes (brome, chlore) ou de leurs dérivés tels que $PBr_3$, $SBr_6$ sur un $\alpha$, $\omega$-chlorohydroxyalcane ou encore à la réaction des halogènes (brome, chlore) ou de leurs dérivés tels que $SOCl_2$ sur un halogènoalcane ou un acide $\omega$-halogènoalcanoïque.

Le brevet anglais 788,349 décrit un procédé de préparation de 1-bromo-4-chlorobutane qui consiste à traiter dans une première étape le THF avec de l'acide chlorhydrique sec en présence de traces de $ZnCl_2$ à une température qui atteint environ 100°C, puis dans une seconde étape à traiter le 4-chloro-1-butanol obtenu précédemment avec du phosphore rouge puis avec du brome sec à une température comprise entre 0°C et -10°C. Le 1-bromo-4-chlorobutane est obtenu avec un rendement d'environ 62 % par rapport au THF mis en oeuvre.

Le brevet US 2,839,574 mentionne un procédé de préparation de 1-bromo-4-chlorobutane qui évite l'emploi de phosphore rouge et de brome ou de $SBr_6$. Ce procédé consiste à traiter du 4-chloro-1-butanol préalablement distillé avec de l'acide bromhydrique gazeux et sec en présence d'un solvant à ébullition et formant un azéotrope avec l'eau formée selon la réaction :

$$Cl(CH_2)_4OH + HBr \rightarrow Br\ (CH_2)_4Cl + H_2O.$$

Le rendement est d'environ 70 %.

Dans la demande de brevet Japonais publiée sous le No JP 5791930, le 1-bromo-4-chlorobutane a été obtenu avec un rendement d'environ 90 % en traitant le 4-chloro-1-butanol fraîchement distillé avec $SBr_6$ formé à partir de soufre et de brome, selon le schéma réactionnel :

$$SBr_6 + 6\ Cl(CH_2)_4OH \rightarrow 6\ Br(CH_2)_4Cl + H_2SO_4 + 2H_2O$$

Dans le cas où l'on fait réagir $SBr_6$ avec un 4-chloro-1-butanol non purifié, provenant notamment d'un mélange de tétrahydrofurane et d'acide chlorhydrique, le rendement en 1-bromo-4-chlorobutane est d'environ 70 %.

D.C. SAYLES et Ed. F. DEGERING (Journal of American Chemistry Society, 71, page 3162, 1949) décrivent une méthode de préparation du 1-bromo-4-chlorobutane en traitant le n-bromobutane avec du chlorure de sulfuryle ($SO_2Cl_2$) en présence de peroxyde de benzoyle, au reflux des réactifs. Le rendement en 1-bromo-4-chlorobutane est de 35 %.

SMUSHKEVICH, YU.I et coll. appliquent la réaction de BORODINE-HUNSDIECKER aux acides $\omega$-chloroalcanoiques (Tr. Mosk. Khim. Technol. Inst. No 61, pp 47-48, 1969).

Ils obtiennent ainsi des $\alpha$,$\omega$-bromochloroalcanes en traitant les acides $\omega$-chloroalcanoiques avec HgO et du brome en milieu $CCl_4$ selon le schéma réactionnel :

$$Cl(CH_2)_n\ COOH + Br_2 \rightarrow Cl(CH_2)_n\ Br + CO_2 + HBr$$

Signalons aussi que HAHN Roger C. (Journal of Organic Chemistry, 53 (6), pp 1331-3, 1988) décrit une méthode de préparation d'$\alpha$,$\omega$-bromochloroalcanes par une réaction d'échange d'halogènes. Ainsi, le 1-bromo-6-chlorohexane est obtenu en chauffant un mélange de n-bromobutane et de 1,6-dichlorohexane en présence de bromure de tétrabutylammonium selon le schéma réactionnel :

$$Br(CH_2)_3CH_3 + Cl(CH_2)_6Cl \rightarrow Br(CH_2)_6Cl + Cl(CH_2)_3CH_3$$

Toutes ces méthodes présentent de nombreux inconvénients. Elles utilisent des composés de départ souvent impurs nécessitant des opérations de purification ainsi que des réactifs coûteux et de manipulation difficile (P + brome , S + brome).

Les rendements en $\alpha$,$\omega$-bromochloroalcanes sont faibles et les produits obtenus contiennent des impuretés dont l'élimination entraîne des manipulations de séparation difficiles et coûteuses.

On a maintenant trouvé un procédé de préparation directe d'α,ω-bromochloroalcane de formule

$$Br(CH_2)_n\,Cl \qquad (I)$$

dans laquelle n représente un nombre entier allant de 3 à 8, à partir d'éther-oxyde cyclique de formule

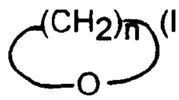 (II)

dans laquelle n a la même signification que dans la formule (I), caractérisé en ce que :

a/ l'on met en contact ledit éther-oxyde cyclique (II), éventuellement additionné d'une quantité d'eau au plus égale à 20 parties en poids pour 100 parties en poids d'éther-oxyde cyclique (II) avec de l'acide chlorhydrique gazeux, puis,
b/ l'on met en contact la phase précédente obtenue en a/ avec de l'acide bromhydrique gazeux.

Selon la présente invention, la quantité d'eau additionnée à l'éther-oxyde cyclique (II) est comprise entre 1 partie et 15 parties en poids pour 100 parties en poids d'éther-oxyde cyclique (II) mis en oeuvre, et, de préférence, une quantité comprise entre 5 et 10 parties en poids.
On ne sortirait pas du cadre de l'invention si l'on effectuait l'étape a/ sans addition d'eau.
A titre d'exemple d'éther-oxyde cyclique de formule (II) utilisable selon la présente invention, on peut citer l'oxyde de 1,3-propylène (oxétane), le tétrahydrofurane, le tétrahydropyrane, l'oxyde de 1,6-hexaméthylène(oxépane), l'oxyde de 1,7-heptaméthylène (oxocane).
Le procédé de la présente invention s'applique tout particulièrement à la préparation du 1-bromo-4-chlorobutane à partir du tétrahydrofurane.
Selon la présente invention, il n'est pas nécessaire d'isoler et/ou de purifier l'α,ω-chlorohydroxyalcane (III) obtenu en a/ par hydrochloration de l'éther-oxyde cyclique (II) selon le schéma réactionnel :

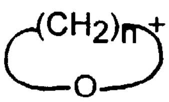 $+ HClgaz \longrightarrow HO(CH_2)nCl$ **A**

(II) (III)

L'étape b/ est effectuée en faisant réagir directement la phase précédente obtenue en a/ -contenant l'intermédiaire (III)- avec de l'acide bromhydrique gazeux pour conduire à l'α,ω-bromochloroalcane (I) selon le schéma réactionnel :

$$HO(CH2)nCl + HBrgaz \rightarrow Br(CH_2)nCl + H_2O \qquad \textbf{B}$$

(III) (I)

L'un des avantages de l'invention apparaît clairement ici ; il n'est pas nécessaire d'isoler et de purifier l'α,ω-chlorohydroxyalcane (III) intermédiaire obtenu en a/ avant de le faire réagir avec l'acide bromhydrique gazeux (étape b/).
Les étapes a/ et b/ du procédé selon l'invention sont effectuées à une température allant de la température ambiante (environ 20° C) à environ 100° C et, de préférence, à une température allant de 40° C à 70° C.
Selon la présente invention, les températures peuvent être identiques ou différentes dans les deux étapes, mais on préfère opérer à une température unique.
Selon la présente invention, on opère dans l'étape a/ avec un rapport molaire HCl gazeux/éther-oxyde cyclique compris entre 1 et 3 et, de préférence, compris entre 1,10 et 2, et dans l'étape b/ avec un rapport molaire HBr gazeux/éther-oxyde cyclique compris entre 1 et 2 et de préférence compris entre 1,20 et 1,60.

La durée de réaction, pour chacune des étapes a/ et b/ peut varier dans de larges limites, mais elle est généralement comprise entre 2 et 30 heures et, de préférence, entre 10 et 25 heures.

La réaction peut être effectuée sous pression atmosphérique.

Selon la présente invention on introduit l'acide chlorhydrique gazeux dans l'éther-oxyde cyclique éventuellement additionné d'eau, puis, l'introduction terminée, on poursuit la réaction en introduisant directement dans le milieu réactionnel précédemment obtenu l'acide bromhydrique gazeux.

L'introduction de HBr gaz terminée on élimine l'eau formée par décantation du milieu réactionnel puis l'α,ω-bromochloroalcane est purifié notamment par distillation sous pression réduite.

La préparation d'α,ω-bromochloroalcane selon le procédé de l'invention présente l'avantage d'être effectué en deux étapes réactionnelles successives dans un même appareillage sans aucune purification ni séparation de l'α,ω-chlorohydroxyalcane intermédiaire.

Le procédé de l'invention présente également l'avantage d'être effectué sans solvant et en absence de catalyseur.

Les rendements en α,ω-bromochloroalcanes sont élevés et les produits obtenus sont aisément purifiables. Dans certains cas ils présentent une pureté suffisante qui leur permet d'être utilisés tel quel.

Les exemples qui suivent illustrent l'invention .

## Exemple 1

Etape a

Dans un réacteur en verre de 1 litre équipé d'une agitation mécanique (ancre), d'une sonde de température, d'une canne d'injection de gaz et surmonté d'un condenseur à eau glycolée (température environ -20° C) sont introduits, sous atmosphère inerte 325 g (4,5 moles) de tétrahydrofurane et 16,2 g d'eau (5 % pondéral).

L'ensemble est chauffé à 55° C puis on injecte, en 14 h, 255 g (7 moles) d'HCl gaz à un débit décroissant allant de 1,5 mol/h à 0,15 mol/h tout en maintenant la température du milieu réactionnel à 60° C.

En fin d'introduction de l'HCl, le brut réactionnel (593 g) contient 2,7 % d'eau, 14 % d'HCl, 76,5 % de 4-chloro-1-butanol, 3 % de 1,4-dichlorobutane et 1,5 % de THF résiduel. Ainsi, la conversion du THF est supérieure à 97 % et le rendement molaire brut en 4-chloro-1-butanol est d'environ 93 % par rapport au THF mis en oeuvre.

Etape b

Après refroidissement à 50° C, on injecte directement dans le milieu réactionnel a/ obtenu précédemment sous agitation 567 g (7 moles) d'HBr gazeux, en 20 h avec un débit décroissant allant de 0,75 mol/h à 0,15 mol/h. Au fur et à mesure de l'introduction de l'HBr, l'HCl précédemment solubilisé dégaze du milieu réactionnel. L'avancement de la réaction est contrôlé par analyse par chromatographie en phase gazeuse (CPG).

En fin d'introduction de l'HBr, après refroidissement à température ambiante et arrêt de l'agitation, le milieu réactionnel décante. On obtient 280 g d'une phase aqueuse jaunâtre contenant 58 % d'HBr et 728 g d'une phase organique incolore comprenant 91 % de 1-bromo-4-chlorobutane, 2,8 % de 1,4-dichlorobutane, 5,8 % de 1,4-dibromobutane et 0,1 % de 4-chloro-1-butanol résiduel d'où un rendement molaire brut en 1-bromo-4-chlorobutane de 92,5 % par rapport au 4-chloro-1-butanol et de 86 % par rapport au THF mis en oeuvre.

Le 1-bromo-4-chlorobutane est alors purifié par distillation sous pression réduite (30 mmHg) avec une colonne adiabatique de 20 plateaux théoriques. Après soutirage d'une fraction de tête comprenant le 4-chloro-1-butanol résiduel et le 1,4-dichlorobutane, on distille 584 g de 1-bromo-4-chlorobutane d'une pureté, déterminée par CPG, de 99,3 %, soit avec un rendement de distillation de 87,5 %. D'où un rendement molaire en 1-bromo-4-chlorobutane distillé de l'ordre de 75 % par rapport au THF mis en oeuvre ($^{Eb}$30 mmHg = 83,5°C).

## Exemple 2

Etape a

Dans le même appareillage que pour l'exemple 1, sont introduits 325 g de tétrahydrofurane et 16,2 g d'eau. L'ensemble est chauffé à 50° C, sous agitation, puis on injecte en 25 h, 365 g d'HCl gaz à un débit de 1,5 mol/h à 0,35 mol/h tout en maintenant la température du milieu réactionnel à 50°C.

On obtient alors 586,5 g d'un brut réactionnel contenant 2,8 % d'eau, 12,5 % d'HCl, 78,5 % de 4-chloro-1-butanol, 2 % de 1,4-dichlorobutane et 1,7 % de THF résiduel. D'où une conversion du THF de l'ordre de 97 % et un rendement molaire brut en 4-chloro-1-butanol de 94 % par rapport au THF initial.

Etape b

On injecte alors directement dans ce milieu réactionnel à 50°C, 6,9 moles d'HBr gaz en 18 h 30 à un débit de 0,7 mol/h à 0,15 mol/h. On obtient après décantation et séparation, 273 g de phase aqueuse acide et 747 g de phase organique comprenant 2,2 % de 1,4-dichlorobutane, 5,5 % de 1,4-dibromobutane et 92,1 % de 1-bromo-4-chlorobutane. D'où un rendement molaire brut en 1-bromo-4-chlorobutane de 94,5 % par rapport au 4-chloro-1-butanol et de 89 % par rapport au THF mis en oeuvre.

**Exemple 3**

On opère dans le même appareillage et selon les mêmes conditions que pour l'exemple 1, excepté que l'étape a/ est effectuée sans ajout d'eau. En fin d'introduction de HCl gazeux, la conversion du THF est de 85 % et le rendement molaire brut en 4-chloro-1-butanol est d'environ de 82 %.

**Exemple 4** (exemple comparatif)

Dans le même appareillage que pour l'exemple 1, sont introduits 108 g de tétrahydrofurane (1,5 moles) et 444 g (4,5 moles) d'une solution aqueuse d'HCl 37 %. Après chauffage, sous agitation, à 65-70°C pendant 5 h, la conversion du THF en 4-chloro-1-butanol est de 78 %. Après 6 h de chauffage supplémentaire, la conversion du THF en 4-chloro-1-butanol reste inférieure à 82 %.
Dans les conditions de cet exemple, la deuxième étape de synthèse du 1-bromo-4-chlorobutane nécessite d'abord une étape de séparation de la phase aqueuse puis une étape de purification du 4-chloro-1-butanol brut intermédiaire pour éviter la formation trop importante de 1,4-dibromobutane à partir du THF résiduel.

## Revendications

1. Procédé de préparation directe d'α,ω-bromochloroalcane de formule

$$Br(CH_2)_n\,Cl \qquad (I)$$

dans laquelle n représente un nombre entier allant de 3 à 8, à partir d'éther-oxyde cyclique de formule

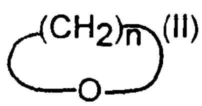
(II)

dans laquelle n a la même signification que dans la formule (I), caractérisé en ce que :

a/ l'on met en contact ledit éther-oxyde cyclique (II), éventuellement additionné d'une quantité d'eau au plus égale à 20 parties en poids pour 100 parties en poids d'éther-oxyde cyclique (II) avec de l'acide chlorhydrique gazeux, puis,
b/ l'on met en contact la phase précédente obtenue en a/ avec de l'acide bromhydrique gazeux.

2. Procédé selon la revendication 1, caractérisé en ce que l'éther-oxyde cyclique (li) est le tétrahydrofurane.

3. Procédé selon l'une des revendications 1 et 2 caractérisé en ce que la quantité d'eau additionnée à l'éther-oxyde cyclique (II) est comprise entre 1 partie et 15 parties en poids pour 100 parties en poids d'éther-oxyde cyclique (II) mis en oeuvre.

4. Procédé selon la revendication 3, caractérisé en ce que la quantité d'eau additionnée à l'éther-oxyde (II) est comprise entre 5 et 10 parties en poids pour 100 parties en poids d'éther-oxyde cyclique (II) mis en oeuvre.

5. Procédé selon l'une des revendications 1 à 4, caractérisé en ce que dans l'étape a/ le rapport molaire HCl gazeux/éther-oxyde cyclique est compris entre 1 et 3 et que, dans l'étape b/ le rapport molaire HBr gazeux/éther-oxyde

cyclique est compris entre 1 et 2.

6. Procédé selon la revendication 5, caractérisé en ce que le rapport molaire HCl gazeux/éther-oxyde cyclique est compris entre 1,10 et 2 et que le rapport molaire HBr gazeux/éther-oxyde cyclique est compris entre 1,20 et 1,60.

7. Procédé selon l'une des revendications 1 à 6, caractérisé en ce que les étapes a/ et b/ sont effectuées à une température allant de la température ambiante à environ 100°C.

8. Procédé selon la revendication 7, caractérisé en ce que les étapes a/ et b/ sont effectuées à une température allant de 40°C à 70°C.

## Patentansprüche

1. Verfahren zur direkten Herstellung eines $\alpha,\omega$-Bromchloralkans der Formel

$$Br(CH_2)_nCl \qquad (I)$$

in der n eine ganze Zahl von 3 bis 8 bedeutet, aus einem cyclischen Ether der Formel

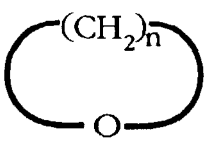

(II)

in der n die gleiche Bedeutung wie in Formel (I) hat, dadurch gekennzeichnet, daß:

a) man den cyclischen Ether (II) mit gasförmigem Chlorwasserstoff zusammengibt, gegebenenfalls unter Zusatz von höchstens 20 Gewichtsteilen Wasser; bezogen auf 100 Gewichtsteile des cyclischen Ethers (II) und
b) man die zuvor bei a) erhaltene Phase mit gasförmigem Bromwasserstoff zusammengibt.

2. Verfahren nach Anspruch 1, dadurch gekennzeichnet, daß der cyclische Ether (II) Tetrahydrofuran ist.

3. Verfahren nach einem der Ansprüche 1 und 2, dadurch gekennzeichnet, daß die dem cyclischen Ether (II) zugegebene Wassermenge zwischen 1 und 15 Gewichtsteilen, bezogen auf 100 Gewichtsteile des eingesetzten cyclischen Ethers (II), ausmacht.

4. Verfahren nach Anspruch 3, dadurch gekennzeichnet, daß die dem cyclischen Ether (II) zugegebene Wassermenge zwischen 5 und 10 Gewichtsteilen, bezogen auf 100 Gewichtsteile des eingesetzten cyclischen Ethers (II), liegt.

5. Verfahren nach einem der Ansprüche 1 bis 4, dadurch gekennzeichnet, daß in der Stufe a) das Molverhältnis von gasförmigem HCl zum cyclischen Ether zwischen 1 und 3 und in Stufe b) das Molverhältnis von gasförmigem HBr zum cyclischen Ether zwischen 1 und 2 liegt.

6. Verfahren riach Anspruch 5, dadurch gekennzeichnet, daß das Molverhältnis von gasförmigem HCl zum cyclischen Ether zwischen 1,10 und 2 und das Molverhältnis von gasförmigem HBr zum cyclischen Ether zwischen 1,20 und 1,60 liegt.

7. Verfahren nach einem der Ansprüche 1 bis 6, dadurch gekennzeichnet, daß die Stufe a) und b) bei einer Temperatur zwischen Umgebungstemperatur und ungefähr 100 °C durchgeführt werden.

8. Verfahren nach Anspruch 7, dadurch gekennzeichnet, daß die Stufe a) und b) bei einer Temperatur zwischen 40 °C und 70 °C durchgeführt werden.

## Claims

**1.** Process for the direct preparation of α,ω-bromochloroalkane of formula

$$Br(CH_2)_nCl \quad (I)$$

in which n represents an integer ranging from 3 to 8, from a cyclic ether of formula

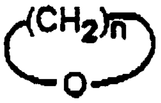 (II)

in which n has the same meaning as in the formula (I), characterized in that:

a) the said cyclic ether (II), optionally with an amount of water added at most equal to 20 parts by weight per 100 parts by weight of cyclic ether (II), is brought into contact with gaseous hydrochloric acid and then
b) the above phase obtained in a) is brought into contact with gaseous hydrobromic acid.

**2.** Process according to Claim 1, characterized in that the cyclic ether (II) is tetrahydrofuran.

**3.** Process according to one of Claims 1 and 2, characterized in that the amount of water added to the cyclic ether (II) is between 1 part and 15 parts by weight per 100 parts by weight of cyclic ether (II) used.

**4.** Process according to Claim 3, characterized in that the amount of water added to the ether (II) is between 5 and 10 parts by weight per 100 parts by weight of cyclic ether (II) used.

**5.** Process according to one of Claims 1 to 4, characterized in that, in the stage a), the gaseous HCl/cyclic ether molar ratio is between 1 and 3 and in that, in the stage b), the gaseous HBr/cyclic ether molar ratio is between 1 and 2.

**6.** Process according to Claim 5, characterized in that the gaseous HCl/cyclic ether molar ratio is between 1.10 and 2 and in that the gaseous HBr/cyclic ether molar ratio is between 1.20 and 1.60.

**7.** Process according to one of Claims 1 to 6, characterized in that the stages a) and b) are carried out at a temperature ranging from room temperature to approximately 100°C.

**8.** Process according to Claim 7, characterized in that the stages a) and b) are carried out at a temperature ranging from 40°C to 70°C.